# EUROPEAN PATENT APPLICATION

(11) **EP 4 574 174 A1**
(43) Date of publication of application: **25.06.2025**
(21) Application number: 23218318.6
(22) Date of filing: 19.12.2023
(51) Int. Cl.: A61K 47/68, A61P 35/00

(54) **NOVEL ANTIBODY-DRUG CONJUGATES COMPRISING AN ANTI-(TCR)-CD3-COMPLEX BINDING ANTIBODY**

(71) Applicant: Elands, Jack, 1190 Forest (BE)
(72) Inventor: Elands, Jack, 1190 Forest (BE)
(74) Representative: Weickmann & Weickmann PartmbB

(57) **Abstract**

The present invention relates to novel antibody-drug conjugates comprising a monoclonal antibody or an antigen-binding fragment thereof wherein the monoclonal antibody or the antigen-binding fragment thereof binds to the (TCR)-CD3 complex.

## Description

The present invention relates to novel antibody-drug conjugates comprising a monoclonal antibody or an antigen-binding fragment thereof wherein the monoclonal antibody or the antigen-binding fragment thereof binds to the (TCR)-CD3 complex.

### BACKGROUND

The T-cell receptor (TCR)-CD3 complex is a critical component of the immune response, playing a pivotal role in the recognition of antigens by T cells. This complex is composed of a diverse ab TCR heterodimer, which is noncovalently associated with the invariant CD3 dimers CD3γε, CD3δε, and CD3ζζ. The αβ TCR heterodimer is responsible for the mediation of recognition of antigenic peptides that are bound to MHC molecules (pMHC). On the other hand, the CD3 molecules are involved in transducing activation signals to the T cells (Mariuzza, Agnihotri, and Orban 2020).

Structurally, the core TCR complex consists of two TCR chains and six cluster of differentiation 3 (CD3) chains. The complex includes additional components such as coreceptors, kinases, and ligands (Shah et al. 2021). The TCR-CD3 complex is recognized as one of the most intricate membrane receptor structures, primarily due to its construction from six distinct chains. This complexity has led to various proposals regarding the arrangement of the receptor subunits, their stoichiometry, and the mechanisms responsible for receptor triggering (Call and Wucherpfennig 2004).

The TCR/CD3 complex functions as a multichain structure that is instrumental in antigen recognition within T cells. Despite the significant amount of genetic, structural, and functional data available, there are still unanswered questions regarding the TCR/CD3 complex. These include the precise number of polypeptides in each TCR/CD3 complex, their interactions, and spatial arrangement, as well as the specific roles they play (Feito et al. 2002).

When an antigen is recognized by the TCR, the CD3 complex transmits signals into the T cell. This process is facilitated by the Immunoreceptor Tyrosine-based Activation Motifs (ITAMs) present in the cytoplasmic tails of the CD3 chains. The phosphorylation of ITAMs leads to the recruitment and activation of various intracellular signaling molecules (Reth 1989).

The TCR/CD3 complex is also pivotal during T cell development in the thymus. The process of T cell maturation involves the selection of T cells based on their TCR specificity, ensuring that they can recognize antigens while being tolerant to selfantigens (von Boehmer 1990)
In terms of assembly, the TCR-CD3 complex forms an octameric structure with a 1:1:1:1 stoichiometry of TCRαβ:CD3γε:CD3δε:CD3ζζ. The assembly of the extracellular domains of the TCR-CD3 is mediated by the constant domains and connecting peptides of TCRαβ, which pack against CD3γε-CD3δε, forming a trimerlike structure proximal to the plasma membrane (Dong et al. 2019).

The TCR/CD3 complex is a target in various immunotherapeutic approaches. For instance, monoclonal antibodies against CD3 (such as Muromonab-CD3) have been used in transplant rejection and autoimmune diseases (Chatenoud et al. 1990).

However, there is still a need for new agents targeting the TCR/CD3 complex for treating TCR/CD3 related diseases.

### SUMMARY OF THE INVENTION

The present invention relates to an antibody-drug conjugate according to formula (I),

AB-M-D (I),

wherein
- AB: is an antibody or antigen-binding fragment thereof that binds to the (TCR)-CD3 complex,
- M: is a linker conjugating AB and D, and
- D: is an active agent or drug.

### ANTIBODIES AB

The antibodies used in the ADCs according to the present invention are antibodies that bind to the (TCR)-CD3 complex (TCRαβ or CD3εy, CD3εδ or CD3ζζ), i.e. anti-TCR or anti-CD3 antibodies.

In a preferred embodiment, the antibody or antigen binding fragment of the invention specifically binds the αchain.

In another preferred embodiment, the antibody or antigen binding fragment of the invention specifically binds the β-chain.

In another preferred embodiment, the antibody or antigen binding fragment of the invention specifically to one of the mutually exclusive expressed T cell receptor β-chain constant domains 1 and 2 (TRBC1 and TRBC2), preferably to both domains.

In another preferred embodiment, the antibody or antigen binding fragment of the invention specifically binds the ε-chain of CD3.

The antibodies of the invention are monoclonal antibodies (mAb) or monoclonal antibody fragments. If not indicated differently, the term "monoclonal" refers to a single species, i.e., single amino acid composition of antibodies or antibody fragments.

The antibodies provided herein show preferably specific binding to TCR or CD3 and no essential or no cross-reactivity to other proteins.

The antigen-binding site of an inventive antibody comprises heavy chain variable domains/regions (VH) and/or antibody light chain variable domains/regions (VL), or pairs of VH/VL.

In particular preferred are the monoclonal antibodies described in WO 2008/079713 and WO 2010/027797.

According to a preferred embodiment, the antibody is an anti-TCR antibody, in particular an anti-TCR antibody comprising a VH domain having the amino acid sequence of SEQ ID NO:10, SEQ ID NO:11, SEQ ID NO:12, SEQ ID NO:13 or SEQ ID NO:14 or an isolated anti-TCR antibody comprising a VL domain having the amino acid sequence of SEQ ID NO:15, SEQ ID NO:16, SEQ ID NO:17, SEQ ID NO:18 or SEQ ID NO: 19, and/or antibodies or antigen-binding fragments thereof which show at least 90 % amino acid sequence identity, preferably 95%, or bind the same epitope as the respective antibody. Anti-TCR antibodies comprising a VH domain having the amino acid sequence of SEQ ID NO:10 and a VL domain having the amino acid sequence of SEQ ID NO:15, or a VH domain having the amino acid sequence of SEQ ID NO:11 and a VL domain having the amino acid sequence of SEQ ID NO:16, or a VH domain having the amino acid sequence of SEQ ID NO:12 and a VL domain having the amino acid sequence of SEQ ID NO:17, or a VH domain having the amino acid sequence of SEQ ID NO:13 and a VL domain having the amino acid sequence of SEQ ID NO:18 and a VH domain having the amino acid sequence of SEQ ID NO:14 and a VL domain having the amino acid sequence of SEQ ID NO:19 are preferred.

An isolated anti-TCR antibody comprising a VH domain having the amino acid sequence of SEQ ID NO:14 and a VL domain having the amino acid sequence of SEQ ID NO:19 is particularly preferred, and/or antibodies or antigen-binding fragments thereof which show at least 90 % amino acid sequence identity, preferably 95%, or bind the same epitope as the respective antibody.

According to another preferred embodiment, the antibody is an anti-CD3 antibody. In a preferred embodiment, the antibody has the binding specificity of the murine monoclonal antibody OKT3 (see, e.g., U.S. Patent Nos. 4,658,019 and 6,113,901), e.g., binds to the same epitope as OKT3 and/or competes for binding (i.e., in an ELISA or immunoprecipitation assay) with OKT3. Preferred is the antibody designated OKT3 (ATCC Accession No. CRL- 8001) and humanized versions of the antibody OKT3, such as OKT3-7 (see the antibodies disclosed in U.S. Patent No. 6,491,916), and/or antibodies or antigen-binding fragments thereof which show at least 90 % amino acid sequence identity, preferably 95%.

Further sequences of humanized OKT3 variable regions comprise the OKT3 light chain (SEQ ID NO:1) and the heavy chain (SEQ ID NO:5) amino acid sequence, the variable domain sequence from the human antibodies chosen as light and heavy chain acceptor framework (SEQ ID NOs :2 and 6, respectively), and the humanized OKT3 variable domain sequences (SEQ ID NO:3, SEQ ID NO:4, SEQ ID NO:7, SEQ ID NO:8 and SEQ ID NO:9) and/or antibodies or antigen-binding fragments thereof which show at least 90 % amino acid sequence identity, preferably 95%, or bind the same epitope as the respective antibody.

Further preferred examples of known antibodies that immunospecifically bind to a CD3 polypeptide include, but are not limited to AMV564, OKT3, HuM291, ChAglyCD3, UCHTI, Leu4, 500A2, CLB-T3/3, BMA030, YTH 12.5 and rat CD3 antibody (See Eissenberg et al., 2018, Blood, 132. Jg., S. 2727, Herald et al, 2005, Diabetes 54:1763-1769; Carpenter et al, 2005, Biol. Blood Marrow Transplant 11 :465-471; Keymeulen et al., 2005, N. Engl. J. Med. 352:26422644; Schwinzer et al., 1992, J. Immunol. 148:1322-1328; Tsoukas et al., 1985, J. Immunol. 135:1719-1723; U.S. Patent No. 6,491,916; Brams et al., 1989, Immunol, 66:348-353; van Lier et al., 1989, Immunol. 68:45-50; Walker et al., 1987, Eur. J. Immunol. 17:1611-1618; Routledge et al., 1991, Eur. J. Immunol. 21 :2717-2725, respectively).

In preferred embodiments, the antibody or antigen binding fragment are sequencemodified to prevent their interaction with Fcy-receptors.

The term "antigen-binding site" denotes the region(s) of an antibody molecule to which a ligand (e.g., the antigen, i.e., TCR or CD3, or antigen fragment of it) actually binds and which is derived from an antibody.

The variable domains/regions denote each of the pair of light and heavy chains, which is involved directly in binding the antibody to the antigen. The variable domain of a heavy chain is abbreviated as "VH" and the variable domain of a light chain is abbreviated as "VL".

An antigen-binding site of an antibody according to the invention can contain six complementarity determining regions (CDRs) which contribute in varying degrees to the affinity of the binding site for the antigen. There are three heavy chain variable domain CDRs (CDR-H1, CDR-H2 and CDR-H3) and three light chain variable domain CDRs (CDR-L1, CDR-L2 and CDR-L3). Also included within the scope of the invention are functional antigen binding sites comprised of fewer CDRs (i.e., where binding specificity is determined by three, four or five CDRs). For example, less than a complete set of 6 CDRs may be sufficient for binding. In some cases, a VH or a VL domain will be sufficient.

According to the present invention, a VH region or the CDRs thereof alone may constitute a complete antigen-binding site. In certain embodiments, the antibody comprises a VH region alone. In other embodiments, the antibody comprises a VH region together with a VL region.

The position of CDRs within a VH or VL region may be defined according to Kabat, et al., Sequences of Proteins of Immunological Interest, 5th Ed. Public Health Service, National Institutes of Health, Bethesda, MD (1991) or the IMGT numbering system, both of which are known to the person skilled in the art. The IMGT numbering has been defined to compare the variable domains whatever the antigen receptor, the chain type, or the species (Lefranc M.-P., "Unique database numbering system for immunogenetic analysis" Immunology Today, 18, 509 (1997); Lefranc M.-P., "The IMGT unique numbering for Immunoglobulins, T cell receptors and Ig-like domains" The Immunologist, 7, 132-136 (1999)). If not stated otherwise, the Kabat system is used herein.

As used herein, the terms "binding" and "specific binding" refer to the binding of the inventive antibody or fragment thereof to an epitope of the antibodies described herein. The measure of the binding strength of an antibody is referred to as affinity. Methods for determining such a binding and/or affinity using in vitro assays are known to the person skilled in the art. According to the present invention, detection with flow cytometry by fluorescence, immuno-histochemistry and/or surface plasmon resonance are described and particularly preferred herein.

The affinity of the binding of an antibody to an antigen is defined by the terms Ka (rate constant for the association of the antibody from the antibody/antigen complex), KD (dissociation constant), and Kdis (KD/Ka).

In certain embodiments, the antibody of the invention may be a chimeric antibody, a multispecific antibody, in particular a bispecific antibody, a human antibody, a humanized antibody, or an antigen-binding fragment thereof.

According to the present invention, a "chimeric" antibody refers to an antibody in which a portion of the heavy and/or light chain is derived from a particular source or species, while the remainder of the heavy and/or light chain is derived from a different source or species.

"Multispecific antibodies" bind two or more different epitopes. The epitopes may be on the same or different antigens. A preferred example of a multispecific antibody is a "bispecific antibody" or an antigen-binding fragment thereof which binds two different epitopes. A special subset of bispecific antibodies is a "biparatopic" antibody or antigen-binding fragment, in which each antigen-binding domain recognizes unique, non-overlapping epitopes on the same target antigen.

In preferred embodiments, the antibody of the invention is a humanized antibody.

The term "humanized antibody" or "humanized version of an antibody" refers to antibodies for which both heavy and light chains are humanized as a result of antibody engineering. A humanized chain is typically a chain in which the V-region amino acid sequence has been changed so that, analyzed as a whole, is closer in homology to a human germ line sequence than to the germline sequence of the species of origin. For example, a murine CDR may be grafted into the framework region of a human antibody to prepare the "humanized antibody." See, e.g., Riechmann, L., et al., Nature 332 (1988) 323-327; and Neuberger, M. S., et al., Nature 314 (1985) 268-270. Other forms of humanized antibodies encompassed by the present invention are those in which the constant region has been additionally modified or changed from that of the original antibody to generate the properties according to the invention. Humanization assessment is based on the resulting amino acid sequence and not on the methodology per se.

Another preferred embodiment refers to human antibodies.

The term "human antibody", as used herein, is intended to include antibodies having variable and constant regions derived from human germ line immunoglobulin sequences. Human antibodies are well-known in the state of the art (van Dijk, M. A., and van de Winkel, J. G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production.

The antibody of the present invention may be of any suitable class. The term "class" refers to the type of constant domain or constant region possessed by its heavy chain. As used herein, "constant domain" or "constant region" denotes the sum of the domains of an antibody other than the variable region. The constant region is not directly involved in binding of an antigen but exhibits various effector functions. The antibody may be of any of the five major classes of antibodies, particularly of the five major classes of human antibodies: IgA, IgD, IgE, IgG, and IgM, or any subclass thereof (isotype), e.g., IgG1, IgG2, IgG3, IgG4, IgA1, and IgA2. The heavy chain constant domains that correspond to the different classes of immunoglobulins are called a, δ, ε, γ and µ, respectively. According to the present invention, an antibody of the class, particularly of the human class IgG, IgA or IgM or a fragment thereof is particularly suitable.

According to a preferred embodiment, an antibody of the invention is selected from class IgG, particularly from the class of human IgG, e.g., of subclass IgG1, IgG2, IgG3 of IgG4, of class IgM, of class IgA or an antigen-binding fragment thereof.

In certain embodiments, the antibody comprises a constant domain, particularly a heavy chain constant domain, more particularly a heavy chain constant domain of the class IgG, particularly of the class human IgG, e.g., of subclass IgG1, IgG2, IgG3 of IgG4, of class IgM, of class IgA, which has a reduced effector function compared to a wild-type sequence of the same subclass, e.g., which has a reduced binding to the Fc receptor. Examples of heavy chain constant domains with reduced effector functions may contain at least one of the mutations D265C, L234A, L235A, P331S, L234F and L235E and/or N297A/S of human IgG, e.g., IgG1 or IgG4 sequences.

An "antigen-binding fragment" of an antibody refers to a molecule comprising a portion of an intact antibody that binds the antigen to which the intact antibody binds. Examples of antibody fragments include but are not limited to Fv, Fab, Fab', Fab'-SH, F(ab')2; diabodies; linear antibodies; single domain antibodies (e.g. VHH), single-chain antibody molecules (e.g., scFv); and multi-specific antibodies formed from antibody fragments. The term also encompasses a fusion protein, e.g., a fusion protein with a non-immunoglobulin peptide or polypeptide, and a conjugate with a non-proteinaceous structure, e.g., a label or a toxin. The terms "antigen-binding fragment of an antibody (thereof)" and "fragment of an antibody (thereof)" may be used interchangeably herein.

The antibody or the antigen-binding fragment may be mono- or multivalent, i.e., it may comprise a single antigen-binding site or multiple antigen-binding sites. For example, Fab fragments have single antigen-binding site, antibodies of the IgG class or Fv or scFv fragments have two antigen-binding sites and antibodies of the IgM class have 5 antigen-binding sites. The term "antibody" also encompasses hetero-specific antibodies, e.g., hetero-bispecific antibodies, which have different antigen-binding sites, particularly antibodies, which are directed to two different epitopes on the antigen. As used herein, "epitope" is a region of an antigen that is bound by an antibody. The term "epitope" includes any polypeptide determinant capable of specific binding to an antibody.

"Percent (%) amino acid sequence identity" with respect to a peptide or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, Alignment for purposes of determining percent amino acid sequence identity can be achieved in various ways that are within the skill in the art, for instance, using publicly available computer software such as BLAST.

Another aspect of the present invention is a combination of at least 2 different monoclonal antibodies or fragments as described herein.

### NUCLEIC ACID MOLECULES, VECTORS AND CELLS

Further, the present invention relates to a nucleic acid molecule, e.g. a DNA molecule, encoding an antibody VH region, or an antibody VL region, or encoding a complete antibody or an antibody fragment as indicated above, a vector or vector system, i.e. a plurality of vectors, comprising said nucleic acid molecule(s) as indicated above, preferably in operative linkage with an expression control sequence, particularly with a heterologous expression control sequence.

Furthermore, the invention relates to a cell comprising a nucleic acid molecule or a vector or vector system as described above. The term "vector," as used herein, refers to a nucleic acid molecule capable of propagating another nucleic acid to which it is linked. According to a preferred embodiment, the vector is an expression vector. An "expression vector" is a vector capable of directing the expression of nucleic acids to which they are operatively linked. Vectors, in particular expression vectors, for the recombinant production of antibodies are well known in the art.

The cell may be a known host cell for producing antibodies or antibody fragments, e.g. a prokaryotic cell such as an E. coli cell, a yeast cell, an insect cell or a mammalian cell, e.g. a CHO cell or a hybridoma cell.

### ACTIVE AGENTSIDRUGS D

An antibody-drug conjugate of the present invention comprises a monoclonal antibody or antigen-binding fragment thereof as defined above, and a drug conjugated to a reactive amino acid residue on the antibody or antigen-binding fragment, e.g., an amino acid residue having a side chain comprising an amino, hydroxy or thiol group, or a reactive group in the antibody glycan structure.

The drug of the antibody-drug conjugate of the invention is preferably selected from topoisomerase I inhibitors, antimitotic agents, cytotoxic agents and/or corticosteroids.

A topoisomerase I inhibitor is a compound, which is capable of forming a ternary complex with topoisomerase I and DNA, thereby preventing DNA re-ligation and introducing DNA strand breaks in the cellular genome. The topoisomerase I inhibitor may be e.g., selected from camptothecin or analogs thereof, indenoisoquinolines and indolocarbazoles.

In a particular embodiment, the topoisomerase-I-inhibitor is camptothecin or an analog thereof, i.e. a compound comprising the pentacyclic basic structure of camptothecin and modified substituents optionally resulting in the presence of a further ring. Specific examples are camptothecin, topotecan, irinotecan, SN-38, belotecan, exatecan including derivatives thereof such as deruxtecan, lurtotecan or atiratecan.

In principle, the drug, e.g. the topoisomerase-I-inhibitor, such as exatecan, may be conjugated to any suitable position of the monoclonal antibody or antigen-binding fragment thereof, particularly to any position, which does not abolish the binding of the antibody to TCR and/or CD3. For example, the drug, e.g. the topoisomerase-I-inhibitor, such as exatecan, may be conjugated to a reactive amino acid residue on the antibody, e.g., an amino acid residue having a side chain comprising an amino, hydroxy or thiol group, or a reactive group in the antibody glycan structure. In particular embodiments, the drug, e.g. the topoisomerase-I-inhibitor, such as exatecan, is conjugated to a reactive thiol group in the side chain of an accessible cysteine residue on the antibody.

In a particular embodiment, the topoisomerase I inhibitor is exatecan or a derivative thereof represented by formula (II)

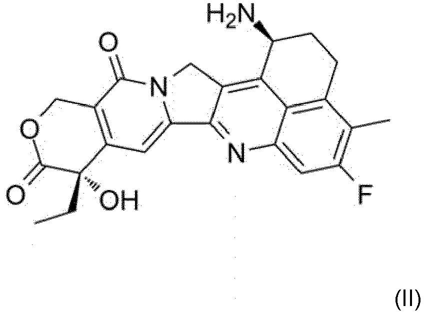

Exatecan is typically conjugated to an antibody via its NH₂ group.

According to another preferred embodiment the topoisomerase-I-inhibitor is belotecan or a derivative thereof being represented by formula (III) 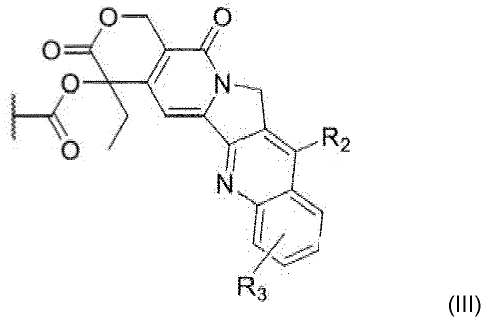 wherein
- R²: is -(CH₂)₂NR¹CH(CH₃)₂
- R¹: is H or -SO₂CH₃
- R³: is H, amino, or C₁-C₆ alkyl, preferably H.

The position where the linker is attached to belotecan or the derivative thereof is indicated by the waved line. Preferably R¹ is H.

Preferred examples of antimitotic agents are auristatin and derivatives thereof, in particular Monomethyl auristatin E (MMAE) or Monomethyl auristatin F (MMAF).

A preferred cytotoxic agent is maytansine and/or derivatives thereof, the so-called maytansinoids.

Maytansine is represented by formula (IV)

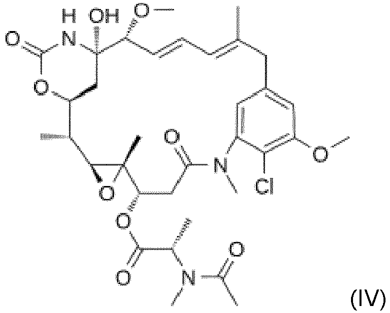

Preferred examples of maytansinoids are Ansamitocin ,Mertansine/emtansine (DM1) or Ravtansine/soravtansine (DM4).

Corticosteroids refer to a class of steroid hormones that are produced in the adrenal cortex of vertebrates, as well as the synthetic analogues of these hormones. Two preferred main classes of corticosteroids, glucocorticoids and mineralocorticoids,

According to the invention in particular preferred corticosteroids are
11-Dehydrocorticosterone (11-oxocorticosterone, 17-deoxycortisone),
11-Deoxycorticosterone (deoxycortone, desoxycortone; 21-hydroxyprogesterone),
11-Deoxycortisol (cortodoxone, cortexolone),
11-Ketoprogesterone (11-oxoprogesterone; Ketogestin),
11β-Hydroxypregnenolone,
11 β-Hydroxyprogesterone (21-deoxycorticosterone),
11β,17α,21-Trihydroxypregnenolone,
17α,21-Dihydroxypregnenolone,
17α-Hydroxypregnenolone,
17α-Hydroxyprogesterone,
18-Hydroxy-11-deoxycorticosterone,
18-Hydroxycorticosterone,
18-Hydroxyprogesterone,
21-Deoxycortisol,
21-Deoxycortisone,
21-Hydroxypregnenolone (prebediolone),
Aldosterone,
Corticosterone (17-deoxycortisol),
Cortisol (hydrocortisone),
Cortisone,
Pregnenolone,
Progesterone
and synthetic analogues thereof.

In particular preferred corticosteroids are Progesteron Cortisol, Corticosterone, Cortisone and Aldosterone and synthetic analogues thereof.

Preferred synthetic analogues of the Progesteron-type are
Flugestone (flurogestone),
Fluorometholone,
Medrysone (hydroxymethylprogesterone) and
Prebediolone acetate (21-acetoxypregnenolone).

Preferred synthetic analogues of the Hydrocortison-type are
Chloroprednisone,
Cloprednol,
Difluprednate,
Fludrocortisone,
Fluocinolone,
Fluperolone,
Fluprednisolone,
Loteprednol,
Methylprednisolone,
Prednicarbate,
Prednisolone,
Prednisone,
Tixocortol and
Triamcinolone,

In certain embodiments, the antibody drug conjugate comprises has a molar drugantibody/antibody fragment ratio (DAR) of greater than 1, i.e., more than one drug molecule is attached to an antibody/antibody fragment. Typically, the conjugate has a DAR of about 2:1 to about 16:1, particularly of about 4:1 to about 10:1 and more particularly of about 6:1 to about 8:1. The DAR may be calculated from a statistical distribution according to known methods.

### LINKER M

The drug can be conjugated to the antibody or antigen-binding fragment thereof via a linker M.

In certain embodiments, the linker is a cleavable linker, i.e., a linker cleavable under physiological conditions, e.g., by physiological enzymes. Specific examples of cleavable linkers are peptide-based linkers, which may be subject to cleavage by a protease, or glycoside-based linkers, which may be subject to cleavage by a glycosidase. Another specific example are branched tandem-cleavage linkers that combine moieties that can be cleaved under different conditions or by different enzymes, e.g. protease-cleavable dipeptide and glycosidase-cleavable sugar moieties. In this way, several identical or different payloads can be connected to the antibody.

In certain embodiments, the linker is a hydrophilic polysarcosine linker e.g., as described by Conilh et at. "Exatecan antibody drug conjugates based on a hydrophilic polysarcosine drug-linker platform" (Pharmaceuticals 14 (2021), 247). Further preferred linkers include linkers comprising at least one ethylene glycol unit, e.g., 1, 2, 3, 4, 5, 6, 7, 8, 9, or 10 or more ethylene glycol units, e.g., the linker of the antibody-drug conjugate MEDI7247, an mcc-triazole spacer-PEG7-x-Lys-PABC glycol linker from Trodelvy^{®}, Further preferred linkers include oligopeptide, particularly di- to decapeptide, e.g., tetrapeptide sequences such as glycine-glycine-phenylalanine-glycine from Enhertu^{®}. Further preferred linkers include highly polar spacers such as an acyl group, carbamoyl group and/or sulfamide group added to at least at least one ethylene glycol unit, e.g., 1, 2, 3, 4, 5, 6, 7, 8, 9, or 10 or more ethylene glycol units, e.g., the linker technology called HydraspaceTM. A particularly preferred linker is a *β-*glucuronide linker.

In certain embodiments, the linker is a hydrophilic polysarcosine linker comprising, e.g., up to 15 sarcosine units, and at least one ethylene glycol unit, e.g., 1, 2, 3, 4, 5, 6, 7, 8, 9, or 10 or more ethylene glycol units, wherein the linker is subject to cleavage by a glycosidase, and particularly subject to cleavage by a glucuronidase.

In certain embodiments, the linker is a hydrophilic polysarcosine linker comprising, e.g., about 8-12 sarcosine units, and at least one ethylene glycol unit, e.g., up to 10 ethylene glycol units, and wherein the linker is subject to cleavage by a glycosidase, and particularly subject to cleavage by a glucuronidase.

In certain embodiments, the linker is a hydrophilic polysarcosine linker comprising 10 sarcosine units, and 2 ethylene glycol units, and wherein the linker is subject to cleavage by a glucuronidase.

A further aspect of the invention relates to a linker-drug conjugate comprising:
(i) a hydrophilic polysarcosine linker comprising, e.g., about 8-12 sarcosine units, and at least one ethylene glycol unit, e.g., 1, 2, 3, 4, 5, 6, 7, 8, 9, or 10 or more ethylene glycol units, wherein the linker is subject to cleavage by a glycosidase, and particularly subject to cleavage by a glucuronidase, and wherein the linker comprises a thiol-reactive group, e.g., a maleimide group capable of reacting with cysteine residues on an antibody or antigen-binding fragment thereof, and
(ii) the drug or a derivative thereof covalently attached to the linker.

In particular embodiments, the linker-drug conjugate comprises:
(i) a hydrophilic polysarcosine linker comprising 10 sarcosine units, and 2 ethylene glycol units, wherein the linker is subject to cleavage by a glycosidase, and particularly subject to cleavage by a glucuronidase, and wherein the linker comprises a maleimide group capable of reacting with cysteine residues on an antibody or antigen-binding fragment thereof, and
(ii) the drug or a derivative thereof covalently attached to the linker.

According to other embodiments, the linker M of the inventive antibody drug conjugate may be characterized by formula (V):

-L-A-E- (V),

wherein
- L: is -(C₁-C₆-alkylene)-(O-CH₂)ₘ-NR¹-,
-(C₁-C₆-alkylene)-NR¹-, or
-(C₁-C₅-alkylene)-NR²C(C=O)-(C₁-C₅-alyklene)-(O-CH₂)^{m}-NR¹-,
wherein R¹ and R² are each independently -H, C₁-C₃-alkyl, in particular methyl, or benzyl,
m is an integer from 1 to 6, in particular 1,
- A: is a peptide comprising 2 to 8 amino acids, preferably 2 to 4 amino acids, wherein A is substituted with at least one polyol, wherein polyol is -(C₁-C₆ alkylene)-X^{a}-Y^{b}, wherein:
X^{a} is -NR^{c}C(=O)- or -C(=O)NR^{c}-;
Y^{b} is -CH₂-(C*₁-C*₅-alkyl)-, each C* substituted with a OH group,
- R^{c}: is -H, C₁-C₆-alkyl, C₁-C₆ fluoroalkyl, C₃-C₆ cycloalkyl, aryl, heteroaryl, or benzyl, and
- E: is -C(=O)-(C₁-C₁₀-alkylene)-Q-, wherein Q is an antibody coupling group.
- L: is -(C₁-C₆-alkylene)-(O-CH₂)ₘ-NR¹-, in particular -(C₁-C₄-alkylene)-O-CH₂-NR¹-, -(C₁-C₆-alkylene)-NR¹-, in particular -(C₁-C₄-alkylene)-NR¹-, or-(C₁-C₅-alkylene)-NR²C(C=O)-(C₁-C₅-alyklene)-(O-CH₂)ₘ-NR¹-, in particular -(C₁-C₅-alkylene)-NR²C(C=O)-(C₁-C₅-alyklene)-O-CH₂-NR¹-, wherein R¹ and R² are each independently -H, C₁-C₃-alkyl, in particular methyl, or benzyl, and m is an integer from 1 to 6, in particular 1.

According to a preferred embodiment L is -(C₁-C₄-alkylene)-O-CH₂-NR¹-, -(C₁-C₄-alkylene)-NR¹-, or -(C₁-C₅-alkylene)-NR²C(C=O)-(C₁-C₅-alyklene)-O-CH₂-NR¹-, wherein R¹ and R² are each independently -H, methyl, or benzyl.

As used herein "alkylene" refers to a saturated linear or branched divalent hydrocarbon radical.

As used herein "cycloalkyl" refers to the radical of a saturated carbocyclic ring. Suitable cycloalkyls include cyclohexyl, cyclopentyl, cyclobutyl and cyclopropyl.

The term "aryl" as used herein, includes substituted or unsubstituted single-ring aromatic groups in which each atom of the ring is carbon. "Aryl" groups include, but are not limited to, phenyl, phenol, aniline, etc. The terms "aryl" also includes "polycyclyl", "polycycle", and "polycyclic" ring systems having two or more rings in which two or more atoms are common to two adjoining rings, e.g., the rings are "fused rings," wherein at least one of the rings is aromatic, e.g., the other cyclic rings can be cycloalkyls, cycloalkenyls, cycloalkynyls, or aromatic rings.

Preferably, the ring is a 5- to 7-membered ring, more preferably a 6-membered ring. Aryl groups include, but are not limited to, phenyl, phenol, aniline, and the like.

"Heteroaryl" as used herein refers to substituted or unsubstituted aromatic single ring structures, preferably 6- to 18-member rings, preferably 5- to 7-membered rings, more preferably 5- to 6-membered rings, whose ring structures include at least one heteroatom (e.g., O, N, or S).

The term "heteroaryl" as used herein, refers to substituted or unsubstituted aromatic single ring structures, preferably 6- to 18-member rings, preferably 5- to 7-membered rings, more preferably 5- to 6-membered rings, whose ring structures include at least one heteroatom (e.g., O, N, or S), preferably one to four or one to three heteroatoms, more preferably one or two heteroatoms. When two or more heteroatoms are present in a heteroaryl ring, they may be the same or different. For examples, "heteroaryl" moieties include, but are not limited to, pyrrole, furan, thiophene, imidazole, oxazole, thiazole, pyrazole, pyridine, pyrazine, pyridazine, quinoline, pyrimidine, indolizine, indole, indazole, benzimidazole, benzothiazole, benzofuran, benzothiophene, cinnoline, phthalazine, quinazoline, carbazole, phenoxazine, quinoline, purine and the like.

Unless specifically stated as "unsubstituted," references to chemical moieties herein are understood to also include substituted variants, i.e. can be substituted with one or more (e.g., 2, 3, 4, 5, 6 or more) substituents. For example, reference to an "alkyl" group or moiety implicitly includes both substituted and unsubstituted variants. Examples of substituents on chemical moieties includes but is not limited to, halogen, hydroxyl, carbonyl (such as carboxyl, alkoxycarbonyl, formyl, or acyl), thiocarbonyl (such as thioester, thioacetate, or thioformate), alkoxyl, alkylthio, acyloxy, phosphoryl, phosphate, phosphonate, amino, amido, amidine, imine, cyano, nitro, azido, sulfhydryl, alkylthio, sulfate, sulfonate, sulfamoyl, sulfonamido, sulfonyl, heterocyclyl, aralkyl, or aryl or heteroaryl moiety.

The amino acids of peptide A may be selected independently. The amino acids may be independently L or D amino acids. According to one preferred embodiment at least one amino acid is an L amino acid. According to other preferred embodiments all amino acids are D or L amino acids. The amino acids alanine (Ala), valine (Val), or glycine (Gly) are in particular preferred.

Suitable examples for a peptide A comprise inter alia -Ala-Ala-, -Gly-Gly-, -Val-Val-, - Val-Ala-, -Ala-Ala-Ala-, -Gly-Ala-Gly-Gly-, -Gly-Gly-Ala-Gly-, -Gly-Val-Gly-Gly-, -Gly-Gly-Val-Gly-, -Gly-Gly-Phe-Gly-, or -Gly-Phe-Gly-Gly- but also flexible linkers like glycine polymers (G)ₙ, glycine-serine polymers (including, for example, (GS)ₙ, (GSGGS)ₙ and (GGGS)ₙ , where n is an integer of at least one, glycine-alanine polymers, alanine-serine polymers, and other flexible linkers known in the art. Such flexible linkers include, but are not limited to -Gly-Gly-Ser-Gly-, -Gly-Gly-Ser-Gly-Gly-, -Gly-Ser-Gly-Ser-Gly-, -Gly-Ser-Gly-Gly-Gly-, -Gly-Gly-Gly-Ser-Gly-, -Gly-Ser-Ser-Ser-Gly-, and the like.

E is -C(=O)-(C₁-C₁₀-alkylene)-Q-, wherein Q is an antibody coupling group. Such antibody coupling groups are well known to the person skilled in the art. Before being coupled, the antibody coupling group is preferably a maleimide group.

After being coupled Q is preferably 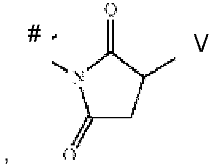 wherein
- #: is the site covalently attached to the remainder of E,
i.e. -C(=O)-(C₁-C₁₀-alkylene)-#, and
- V: is as herein defined.

Corresponding coupling reactions are well known to the person skilled in the art.

According to a preferred embodiment, Y^{b} is C₅-alkyl substituted with 5 OH groups.

According to a further preferred embodiment the at least one polyol is 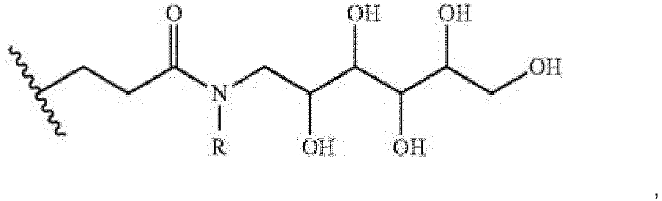 wherein R is H or methyl.

According to another embodiment linker M is as shown in formula (VI) 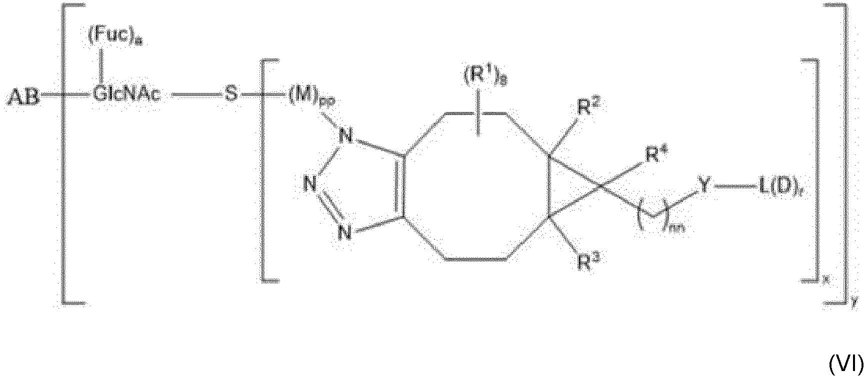 wherein
AB is the antibody or functional fragment thereof as shown in formula (I) and herein defined,
Fuc is fucose;
GlcNAc is N-acetylglucosamine;
S is a sugar or sugar derivative;
M is -N(H)C(O)CH₂-, -N(H)C(O)CF₂-, -CH₂-, -CF₂- or a 1,4-phenylene containing 0-4 fluorine substituents, preferably 2 fluorine substituents which are preferably positioned on C2 and C6 or on C3 and C5 of the phenylene;
R¹ is independently selected from the group consisting of hydrogen, halogen, -OR⁸, -NO₂, -CN, S(O)₂R⁸, C₁-C₂₄ alkyl groups, C₆-C₂₄ (hetero)aryl groups, C₇-C₂₄ alkyl(hetero)aryl groups and C₇-C₂₄ (hetero)arylalkyl groups, preferably hydrogen, and wherein the alkyl groups, (hetero)aryl groups, alkyl(hetero)aryl groups and (hetero)arylalkyl groups are optionally substituted, wherein two substituents R⁸ may be linked together to form an annelated cycloalkyl or an annelated (hetero)arene substituent, and wherein R⁸ is independently selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups;
R² and are R³ are independently selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups, preferably hydrogen;
R⁴ is selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups, preferably hydrogen, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted;
Y is O, S or NR⁷, wherein R⁷ is independently selected from the group consisting of hydrogen, halogen, -OR⁸, -NO₂, -CN, S(O)₂R⁸, C₁-C₂₄ alkyl groups, C₆-C₂₄ (hetero)aryl groups, C₇-C₂₄ alkyl(hetero)aryl groups and C₇-C₂₄ (hetero)arylalkyl groups and wherein the alkyl groups, (hetero)aryl groups, alkyl(hetero)aryl groups and (hetero)arylalkyl groups are optionally substituted, wherein two substituents R⁸ may be linked together to form an annelated cycloalkyl or an annelated (hetero)arene substituent and wherein R⁸ is independently selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups;
L is a linking group, preferably a sulfamide based linking group,
a is 0 or 1;
x is 1 or 2, preferably 1;
y is 1, 2, 3 or 4, preferably 1,
r is 1, 2, 3, 4, 5, 6, 7, 8, 9, 10, 11, 12, 13, 14, 15, 16, 17, 18, 19 or 20, preferably 1 or 2;
nn is 1, 2, 3, 4, 5, 6, 7, 8, 9 or 10, preferably 1 or 2, in particular 1;
pp is 0 or 1, preferably 1;
D is is the drug or active agent as shown in formula (I) and herein defined.

The general term "sugar" is herein used to indicate a monosaccharide, for example glucose (Glc), galactose (Gal), mannose (Man) and fucose (Fuc).

The term "sugar derivative" is herein used to indicate a derivative of a monosaccharide sugar, i.e. a monosaccharide sugar comprising substituents and/or functional groups.

Examples of a sugar derivative include amino sugars and sugar acids, e.g. glucosamine (GlcNH₂), galactosamine (GalNH₂) N-acetylglucosamine (GIcNAc), N-acetylgalactosamine (GaINAc), sialic acid (Sia) which is also referred to as N-acetylneuraminic acid (NeuNAc), and N-acetylmuramic acid (MurNAc), glucuronic acid (GlcA) and iduronic acid (IdoA). GalNAc is in particular preferred.

GIcNAc moieties as shown in Formula (I) are preferably present at a native N-glycosylation site in the Fc-fragment anti-(TCR) or anti-CD3 antibody or functional fragment thereof. Preferably said GlcNAc moieties are attached to an asparagine amino acid in the region 290-305 of the antibody. In a further preferred embodiment, the antibody is an IgG type antibody, and depending on the particular IgG type antibody, said GlcNAc moieties are present on amino acid asparagine 297 (Asn297 or N297) of the antibody. Of course, other attachment sites, i.e. "attachment amino acids" are also possible.

According to a preferred embodiment b is 1.

According to a preferred embodiment x is 1.

According to a preferred embodiment R¹, R², R³ and R⁴ are hydrogen.

According to a preferred embodiment nn is 1 or 2, in particular 1.

According to a preferred embodiment Y is O. If Y is O, b is preferably 0.

According to a preferred embodiment M is -CH₂-, particular pp being 1.

According a preferred embodiment x is 1, and R¹, R², R³ and R⁴ are hydrogen.

According a preferred embodiment x is 1, and R¹, R², R³ and R⁴ are hydrogen and nn is 1.

According a preferred embodiment x is 1, R¹, R², R³ and R⁴ are hydrogen and nn is 1 and Y is O.

According a preferred embodiment x is 1, R¹, R², R³ and R⁴ are hydrogen, Y is O, nn is 1 and M is -CH₂- with pp being 1.

The compounds disclosed in this description and in the claims may comprise one or more asymmetric centres, and different diastereomers and/or enantiomers may exist of the compounds. The description of any compound in this description and in the claims is meant to include all diastereomers, and mixtures thereof. In addition, the description of any compound in this description and in the claims is meant to include both the individual enantiomers, as well as any mixture of the enantiomers and/or diastereomers. When the structure of a compound is depicted as a specific enantiomer, it is to be understood that the invention of the present application is not limited to that specific enantiomer.

According to a preferred embodiment L comprises a group according to formula (VII) 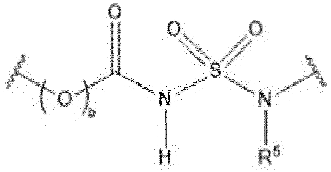 wherein b is 0 or 1;
- R⁵: is selected from the group consisting of hydrogen, C₁-C₂₄ alkyl groups, C₃-C₂₄ cycloalkyl groups, C₂-C₂₄ (hetero)aryl groups, C₃-C₂₄ alkyl(hetero)aryl groups and C₃-C₂₄(hetero)arylalkyl groups, preferably hydrogen,
the C₁-C₂₄ alkyl groups, C₃-C₂₄ cycloalkyl groups, C₂-C₂₄ (hetero)aryl groups, C₃-C₂₄ alkyl(hetero)aryl groups and C₃-C₂₄(hetero)arylalkyl groups optionally substituted and/or optionally interrupted by one or more heteroatoms selected from O, S or NR⁶, wherein R⁶ is hydrogen or C₁-C₄ alkyl, preferably hydrogen; wherein D is optionally connected to N via a spacer moiety.

A "spacer moiety" as used herein refers to a moiety that spaces (i.e. provides distance between) and covalently links together two (or more) parts of a linker. The linker may be part of e.g. a linker-construct, the linker-conjugate or a bioconjugate as herein defined.

If Y is O, b is preferably 0.

R⁵ is preferably hydrogen.

According a preferred embodiment x is 1, R¹, R², R³ and R⁴ are hydrogen, Y is O, nn is 1 and M is -CH2- with pp being 1, and L comprises Formula (VII), wherein in particular preferred R¹, R², R³, R⁴ and R⁵ are hydrogen.

According to a preferred embodiment the spacer moiety is represented by Formula (VIII)

Q-(L¹)ₙ-(CO)-(L²)ₒ-(L³)ₚ-(L⁴)_{q}- ,

wherein
- Q: is -(W)ₖ-(A)_{d}-(B)ₑ-(A¹)_{f}- , wherein
- W: is -OC(O)-, -C(O)O-, -C(O)NH-, -NHC(O)-, -OC(O)NH-,-NHC(O)O-, -C(O)(CH₂)ₘC(O)-, -C(O)(CH₂)ₘC(O)NH-, or - (4-Ph)CH₂NHC(O)(CH₂)ₘC(O)NH-, wherein m is an integer in the range 0-10;
- A: is of Formula (II) as defined above;
- A¹: is of Formula (II) as defined above with R⁵ being preferably (L¹)ₙ-(CO)-(L²)ₒ-(L³)ₚ-(L⁴)_{q}-,
- B: is a (-CH₂-CH₂-O-)_{g} or a -O-CH₂-CH₂- moiety, or (B)_{g} is a -(CH₂-CH₂-O)_{g}-CH₂CH₂- moiety, wherein g is an integer in the range 1 - 10, preferably being 1 or 2;
- k: is 0 or 1, with the proviso that if k is 1 then d is 0;
- d: is 0 or 1;
- e: is an integer in the range 1 - 10;
- f: is 0 or 1;

- L¹: is a -CH₂-CH₂-O-, -CH₂-CH₂-O-C(O)-, -O-CH₂-CH₂-, or -(CH₂-CH₂-O)ₙ₁-CH₂-CH₂- moiety, wherein n1 is an independently selected integer in the range of 1-10, preferably -CH₂-CH₂-O- or -CH₂-CH₂-O-C(O)-,
- L²: is a peptide spacer, preferably a dipeptide wherein L² is represented by general structure (IX): 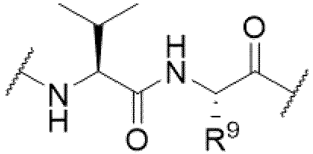 wherein R⁹ is hydrogen, CH₃ or CH₂CH₂CH₂NHC(O)NH₂, preferably R⁹ is hydrogen;
- L³: is self-immolative spacer, preferably a paraaminobenzyloxycarbonyl (PABC) derivative according to structure (X) 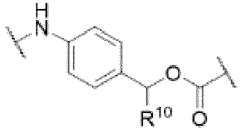 wherein R¹⁰ is hydrogen, R¹¹ or C(O)R¹¹,
wherein R¹¹ is selected from C₁-C₂₄ (hetero)alkyl groups, C₃-C₁₀ (hetero)cycloalkyl groups, C₂-C₁₀ (hetero)aryl groups, C₃-C₁₀ alkyl(hetero)aryl groups and C₃-C₁₀ (hetero)arylalkyl groups, which are optionally substituted and optionally interrupted by one or more heteroatoms selected from O, S and NR¹² wherein R¹² is independently selected from the group consisting of hydrogen and C₁-C₄ alkyl groups, R¹⁰ preferably being hydrogen or C(O)R¹¹ wherein R¹¹ is preferably 4-methyl-piperazine or morpholine, most preferably wherein R¹⁰ being hydrogen;
- L⁴: is an aminoalkanoic acid spacer according to the structure -N-(Cₓ-alkylene)-C(O)-, wherein x is an integer in the range 1-10, or L⁴ is an ethyleneglycol spacer according to the structure -N-(CH₂-CH₂-O)ₓ₁-(CH₂)ₓ₂-C(O)-, wherein x1 is an integer in the range 1-10 and x2 is an integer in the range 1-3; and
n, o, p, q are independently selected from 0 or 1.

Preferably at least one of d and f is 1.

According to a preferred embodiment R⁵ of A¹ is of -(L¹)ₙ-(CO)-(L²)ₒ-(L³)ₚ-(L⁴)_{q}-as defined herein, i.e. the corresponding N atom is substituted by two residues of Formula

-(L¹)ₙ-(CO)-(L²)ₒ-(L³)ₚ-(L⁴)_{q}-

as defined above. The individual variables may be selected independently. According to a preferred embodiment the residues of Formula -(L¹)ₙ-(CO)-(L²)ₒ-(L³)ₚ-(L⁴)_{q}- are identical.

If Y is O, k is preferably 0, in particular b and k are 0.

"Peptide spacers" as used herein are known by the skilled person preferably comprising 2-5 amino acids, more preferably a dipeptide or tripeptide spacer, most preferably a dipeptide spacer. L² may be selected from Val-Cit, Val-Ala, Val-Lys, Val-Arg Phe-Cit, Phe-Ala, Phe-Lys, Phe-Arg, Ala-Lys, Leu-Cit, Ile-Cit, Trp-Cit, Ala-Ala-Asn, Ala-Asn, more preferably Val-Cit, Val-Ala, Val-Lys, Phe-Cit, Phe-Ala, Phe-Lys, Ala-Ala-Asn, more preferably Val-Cit, Val-Ala, Ala-Ala-Asn. In the particular preferred embodiment the peptide spacer L2 is represented by general formula (IX).

According to a preferred embodiment R⁹ is hydrogen.

According to a preferred embodiment R¹⁰ is hydrogen.

According to a preferred embodiment x is 1, R¹, R², R³ and R⁴ are hydrogen and L² is represented by general structure (IX).

According to a preferred embodiment x is 1, R¹, R², R³ and R⁴ are hydrogen and L² is represented by general structure (IX) and R⁹ is hydrogen.

According to a preferred embodiment x is 1, R¹, R², R³ and R⁴ are hydrogen and L² is represented by general structure (IX), R⁹ is hydrogen and Y is O.

According to a preferred embodiment x is 1, R¹, R², R³ and R⁴ and are hydrogen and L³ is a PABC derivative as herein defined.

According to a preferred embodiment x is 1, R¹, R², R³ and R⁴ and are hydrogen, Y is O, L² is represented by general structure (IX), and L³ is a PABC derivative as herein defined.

According to a preferred embodiment x is 1, R¹, R², R³, R⁴ and R⁹ are hydrogen.

According to a preferred embodiment x is 1, R¹, R², R³, R⁴ and R¹⁰ are hydrogen.

According to a preferred embodiment x is 1, R¹, R², R³, R⁴, R⁹ and R¹⁰ are hydrogen.

According to a preferred embodiment x is 1, R¹, R², R³, R⁴ and R¹⁰ are hydrogen and Y is O.

According to a preferred embodiment x is 1, R¹, R², R³, R⁴ and R¹⁰ are hydrogen, Y is O and L³ is a PABC derivative as herein defined.

According to a preferred embodiment x is 1, R¹, R², R³, R⁴ and R¹⁰ are hydrogen, Y is O, L³ is a PABC derivative as herein defined and M is -CH₂-with pp being 1.

According to a preferred embodiment x is 1, R¹, R², R³, R⁴, R⁹ and R¹⁰ are hydrogen and Y is O.

According to an especially preferred embodiment x is 1, R¹, R², R³, R⁴, R⁹ and R¹⁰ are hydrogen, L² is represented by general structure (XV), Y is O and L³ is a PABC derivative as herein defined.

According to an especially preferred embodiment x is 1, R¹, R², R³, R⁴, R⁹ and R¹⁰ are hydrogen, L² is represented by general structure (XV), Y is O, L³ is a PABC derivative as herein defined and M is -CH₂- with pp being 1.

A "self-immolative group" as used herein is a part of a linker in an antibody-drug conjugate with a function to conditionally release a free drug at the site targeted by the ligand unit. The activatable self-immolative moiety may comprise an activatable group (AG) and a self-immolative spacer unit. Upon activation of the activatable group, for example by enzymatic conversion of an amide group to an amino group or by reduction of a disulfide to a free thiol group, a self-immolative reaction sequence is initiated that leads to release of free drug by one or more of various mechanisms. Alternatively, the self-immolative group is not an inherent part of the chemical spacer, but branches off from the chemical spacer connecting the antibody and the payload.

The linker described herein is, for example, described in WO 2021/144313, WO 2016/053107 as well as WO 2017/137456. Methods for attaching the described linkers to antibodies are, for example, described in WO 2011/136645, WO 2022/2022049211, WO 2014/065661, WO 2016/270186, WO 2017/137459 and WO 2021/015622.

The linker-drug conjugates described herein are particularly suitable for attachment to an anti-(TCR) or anti-CD3 antibody as described herein. It should be noted, however, that the linker-drug conjugates are also suitable for attachment to any antibody or antigen-binding fragment thereof, e.g., an antibody binding to a tumor antigen or an antigen-binding fragment thereof.

The antibody-drug conjugate of the present invention may be prepared by known methods.

The antibody or the antigen-binding fragment thereof may be produced in a suitable host cell comprising a nucleic acid molecule, e.g., a DNA molecule, encoding an antibody VH region, or an antibody VL region, or encoding a complete antibody or an antibody fragment, or a vector or vector system, i.e. a plurality of vectors, comprising said nucleic acid molecule(s), preferably in operative linkage with an expression control sequence, particularly with a heterologous expression control sequence. The host cell may be any known host cell for producing antibodies or antibody fragments, e.g., a prokaryotic cell such as an E. coli cell, a yeast cell, an insect cell, or a mammalian cell, e.g., a CHO cell or a hybridoma cell.

The antibody or antigen-binding fragment thereof may be reacted with a linker-drug conjugate to obtain the antibody-drug conjugate. The linker-drug conjugate comprises a drug molecule, e.g., having attached thereto a suitable linker, wherein the linker comprises a reactive group capable of reacting with desired attachment positions on the antibody or antigen-binding fragment thereof. For attachment to cysteine residues, the linker-drug conjugate comprises a thiol-reactive group, e.g., a maleimide group.

In another specific embodiment, binding of the conjugating linker to the antibody can take place, for example, via aldehyde groups that may have been previously introduced into the antibody. For this purpose, the ligand may include a Hydrazineiso-Picted-Spengler (HIPS) chemistry, the binding of which to the aldehyde group leads to the formation of a stable C-C bond. This HIPS chemistry can be combined with any of the linker types described above.

### PHARMACEUTICAL COMPOSITIONS

A further aspect of the present invention is a pharmaceutical composition comprising an active agent, which is an antibody-drug conjugate as herein described and a pharmaceutically acceptable carrier and/or excipient.

Examples of suitable carriers and excipients for formulating antibodies and antibody drug conjugates include saline and aqueous buffer solutions and are well known in the art. Typically, the pharmaceutical composition is adapted for parenteral administration, e.g., for subcutaneous, intramuscular, or intravenous injection or by infusion. In further embodiments, the pharmaceutical composition is adapted for local administration, e.g., for intravesical instillation into the bladder.

Depending on the stage and the severity of the disorder, the pharmaceutical composition may be administered once or several times in a therapeutically effective dose to a subject in need thereof, particularly to a human subject. For example, it may be administered once or several times daily, each second day, two times weekly or weekly for a suitable period, e.g., of at least one week, or at least one month.

### MEDICAL APPLICATIONS

According to a further aspect of the invention the antibody-drug conjugate or pharmaceutical composition as described above is used in medicine, including human and veterinary medicine, particularly in human medicine.

In a particular embodiment, the antibody-drug conjugate or pharmaceutical composition as described above is used in a method for the prevention and/or treatment of a (TCR)-CD3-complex associated disorder such as cancer, in particular specifically targeting T lymphocytes in the treatment of T-cell malignancies, inflammatory diseases, autoimmune diseases, immunosuppressive induction therapy for transplant engraftment and/or immunosuppressive therapy in graft versus host disease.

The cancer to be prevented and/or treated may be any kind of cancer, wherein the term "cancer" is used herein to refer to proliferative diseases, in particular T-cell mediated lymphoproliferative disorders. T-cell mediated lymphoproliferative disorders are a group of diseases characterized by the abnormal growth and proliferation of T lymphocytes, a type of white blood cell vital for the immune response. These disorders can range from relatively benign to highly aggressive malignancies

The cancer to be prevented and/or treated according to the present invention is preferably selected from the group consisting of T-cell lymphomas, T-cell leukemia, T-cell Large Granular Lymphocytic Leukemia and/or Angioimmunoblastic T-cell Lymphoma (AITL).

Autoimmune diseases are conditions where the immune system mistakenly attacks the body's own cells and tissues. In a healthy immune system, the body defends against foreign substances like bacteria and viruses. However, in autoimmune diseases, this system malfunctions, leading to inflammation and damage in various parts of the body. These diseases can affect almost any part of the body, including the heart, brain, nerves, muscles, skin, eyes, joints, lungs, kidneys, glands, the digestive tract, and blood vessels.

Autoimmune diseases involving T lymphocytes are a group of disorders where the immune system, particularly T lymphocytes (a type of white blood cell), mistakenly attacks the body's own cells and tissues. These diseases can vary widely in their symptoms, severity, and the tissues they affect.

The autoimmune disease to be prevented and/or treated according to the present invention is preferably selected from the group consisting Type 1 Diabetes, Multiple Sclerosis (MS), Rheumatoid Arthritis (RA), Psoriasis, Systemic Lupus Erythematosus (SLE) and/or Inflammatory Bowel Diseases (like Crohn's Disease and Ulcerative Colitis).

In therapeutic applications, the active agent is administered in an effective amount to a subject in need thereof, particularly to a human subject. The dose will depend on the specific type of agent, e.g., type of antibody or antibody fragment, the type of disease, and the mode of administration, e.g., locally or systemically.

In the prevention and/or treatment of cancer, a therapeutic dose of an antibody or antibody drug conjugate is typically from about 0.3 mg/kg to about 10 mg/kg.

The antibody-drug conjugate may be administered alone or together with a further active agent, which may be selected from chemotherapeutic agents, e.g., antimetabolites, alkylating agents, intercalating agents, or anti-mitotic agents), inhibitors of specific kinases e.g., tyrosine kinase inhibitors, serine/threonine kinase inhibitors or phosphoinositide kinase inhibitors, immunotherapeutic compounds, e.g., immune checkpoint inhibitors, CAR-T cells, therapeutic vaccines or oncolytic viruses.

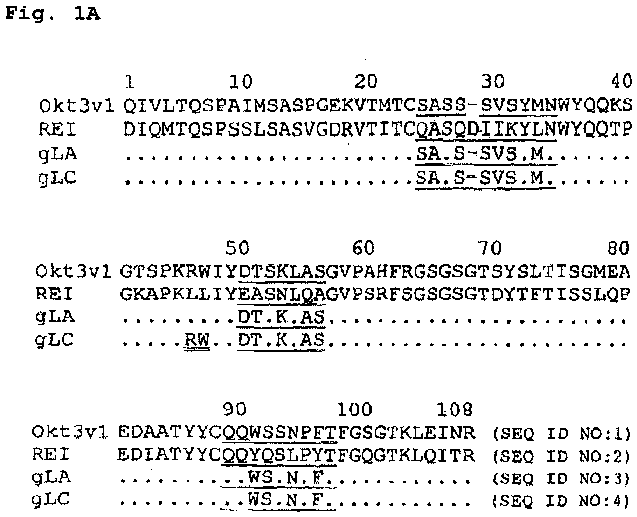

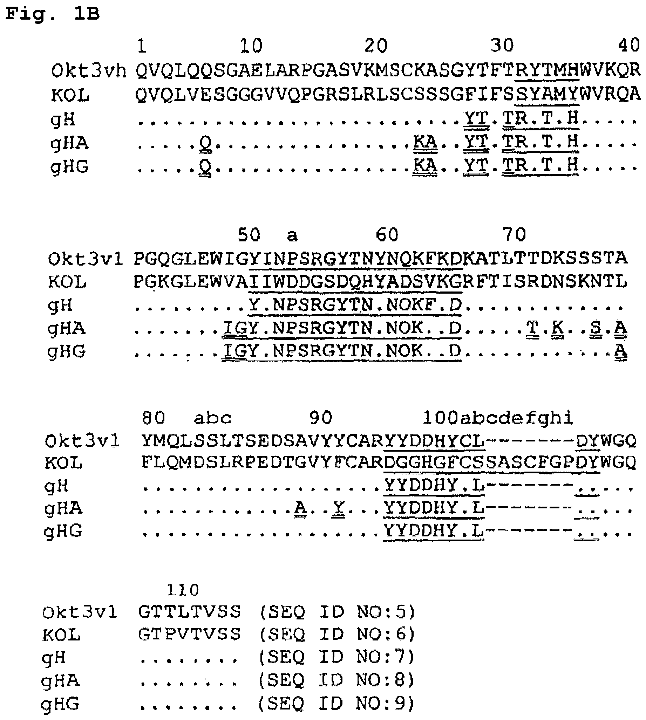

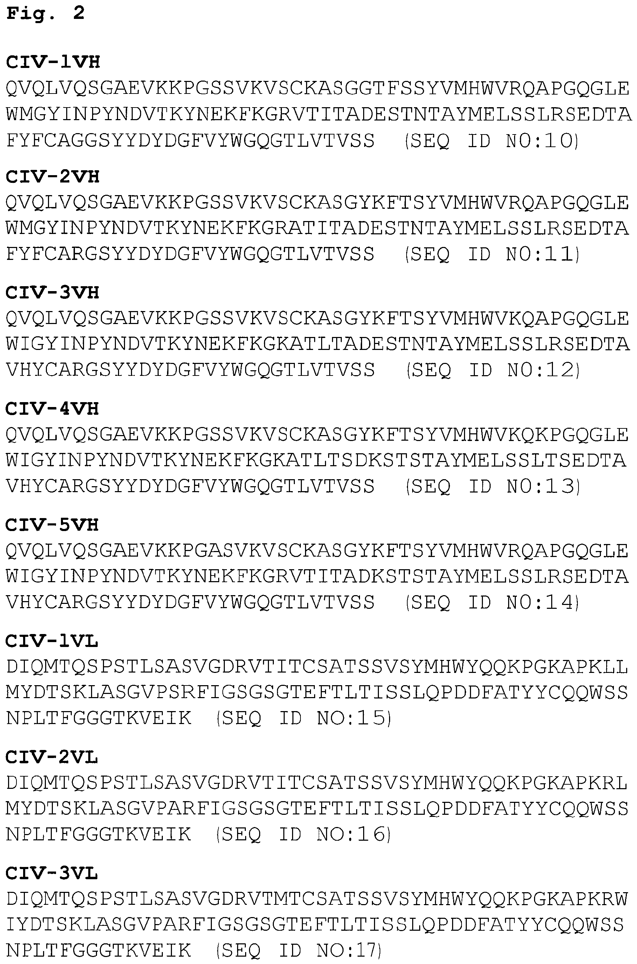

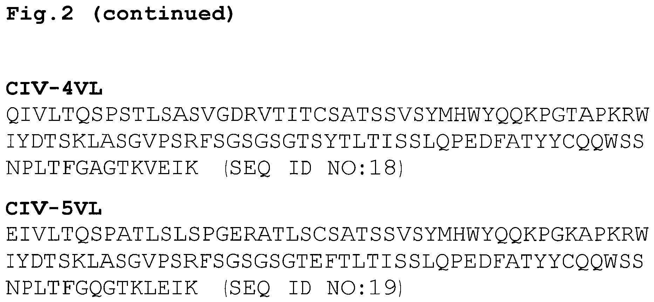

## Claims

1. An antibody-drug conjugate of Formula I, or a pharmaceutically acceptable salt thereof,
AB-M-D (I)
wherein
AB is an antibody or antigen-binding fragment thereof that binds the (TCR)-CD3 complex,
M is a linker conjugating AB and D, and
D is an drug or active agent.

2. The antibody-drug conjugate of claim 1,
wherein the antibody or antigen-binding fragment thereof binds TCR.

3. The antibody-drug conjugate of claim 2,
wherein the antibody or antigen-binding fragment comprises a VH domain having the amino acid sequence of SEQ ID NO:2, SEQ ID NO:3, SEQ ID NO:4, SEQ ID NO:5 or SEQ ID NO:6 and/or a VL domain having the amino acid sequence of SEQ ID NO:8, SEQ ID NO:9, SEQ ID NO: 10, SEQ ID NO: 11 or SEQ ID NO: 12,
and/or antibodies or antigen-binding fragments thereof which show at least 90 % amino acid sequence identity, preferably 95%, or bind the same epitope as the respective antibody.

4. The antibody-drug conjugate of claim 1,
wherein the antibody or antigen-binding fragment thereof binds CD3.

5. The antibody-drug conjugate of claim 4,
wherein the antibody or antigen-binding fragment thereof is a humanized version of OKT3, Leu-4, 500A2, CLB-T3/3, M291, YTH 12.5 or BMA030, and/or antibodies or antigen-binding fragments thereof which show at least 90 % amino acid sequence identity, preferably 95%, or bind the same epitope as the respective antibody.

6. The antibody-drug conjugate of any of the preceding claims,
wherein the antibody or antigen-binding fragment thereof is selected from a chimeric antibody, a multispecific antibody, in particular a bispecific antibody, a human antibody, a humanized antibody, or an antigen-binding fragment thereof.

7. The antibody-drug conjugate of any of the preceding claims,
wherein the antibody or antigen-binding fragment thereof is an antibody selected from an antibody of class IgG, e.g., of subclass IgG1, IgG2, IgG3 of IgG4, of class IgM, of class IgA or an antigen-binding fragment thereof, or a single-chain antibody, or an antibody Fv fragment, wherein the antibody optionally has a heavy chain constant domain having a reduced effector function, e.g., which has a reduced binding to the Fc receptor.

8. The antibody-drug conjugate of any of the preceding claims,
wherein M is a cleavable linker, and/or wherein M is a hydrophilic polysarcosine linker, a hydrophilic linker comprising at least one ethylene glycol unit, a linker comprising a highly polar spacer, or an oligopeptide linker.

9. The antibody-drug conjugate of any of the preceding claims,
wherein M is a hydrophilic polysarcosine linker comprising, e.g., about 8-12 sarcosine units, and at least one ethylene glycol unit, e.g., 1, 2, 3, 4, 5, 6, 7, 8, 9, or 10 or more ethylene glycol units, or
wherein M is a linker comprising Val-Ala or GGFG and optionally at least one ethylene glycol unit, e.g., 1, 2, 3, 4, 5, 6, 7, 8, 9, or 10 or more ethylene glycol units, in particular a Val-Ala or GGFG linker with 8 ethylene glycol units.

10. The antibody-drug conjugate of any of the preceding claims,
wherein the linker is a hydrophilic polysarcosine linker comprising 10 sarcosine units and 2 ethylene glycol units.

11. The antibody-drug conjugate of any of the preceding claims,
wherein the linker is subject to cleavage by a glycosidase, and particularly subject to cleavage by a glucuronidase.

12. The antibody-drug conjugate of any of the preceding claims,
which is of formula (VI) 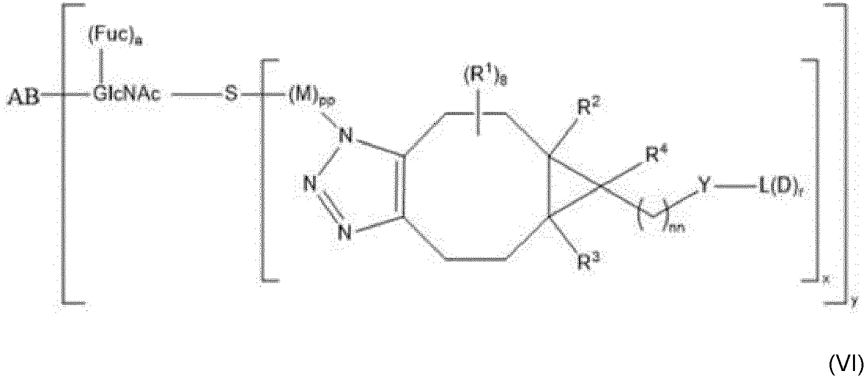 wherein
AB is the antibody or functional fragment thereof as defined according to any of the preceding claims,
Fuc is fucose;
GlcNAc is N-acetylglucosamine;
S is a sugar or sugar derivative;
M is -N(H)C(O)CH₂-, -N(H)C(O)CF₂-, -CH₂-, -CF₂- or a 1,4-phenylene containing 0-4 fluorine substituents, preferably 2 fluorine substituents which are preferably positioned on C2 and C6 or on C3 and C5 of the phenylene;
R¹ is independently selected from the group consisting of hydrogen, halogen, -OR⁸, -NO₂, -CN, S(O)₂R⁸, C₁-C₂₄ alkyl groups, C₆-C₂₄ (hetero)aryl groups, C₇-C₂₄ alkyl(hetero)aryl groups and C₇-C₂₄ (hetero)arylalkyl groups, preferably hydrogen, and wherein the alkyl groups, (hetero)aryl groups, alkyl(hetero)aryl groups and (hetero)arylalkyl groups are optionally substituted, wherein two substituents R⁸ may be linked together to form an annelated cycloalkyl or an annelated (hetero)arene substituent, and wherein R⁸ is independently selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups;
R² and R³ are independently selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups, preferably hydrogen;
R⁴ is selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups, preferably hydrogen, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted;
Y is O, S or NR⁷, wherein R⁷ is independently selected from the group consisting of hydrogen, halogen, -OR⁸, -NO₂,-CN, S(O)₂R⁸, C₁-C₂₄ alkyl groups, C₆-C₂₄ (hetero)aryl groups, C₇-C₂₄ alkyl(hetero)aryl groups and C₇-C₂₄ (hetero)arylalkyl groups and wherein the alkyl groups, (hetero)aryl groups, alkyl(hetero)aryl groups and (hetero)arylalkyl groups are optionally substituted, wherein two substituents R⁸ may be linked together to form an annelated cycloalkyl or an annelated (hetero)arene substituent and wherein R⁸ is independently selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups;
L is a linking group, preferably a sulfamide based linking group,
a is 0 or 1;
x is 1 or 2, preferably 1;
y is 1, 2, 3 or 4, preferably 1,
r is 1, 2, 3, 4, 5, 6, 7, 8, 9, 10, 11, 12, 13, 14, 15, 16, 17, 18, 19 or 20, preferably 1 or 2;
nn is 1, 2, 3, 4, 5, 6, 7, 8, 9 or 10, preferably 1 or 2, in particular 1;
pp is 0 or 1, preferably 1;
D is is a drug.

13. The antibody-drug conjugate of any of the preceding claims,
wherein the drug or active agent D is selected from the group consisting of topoisomerase I inhibitors (in particular camptothecin derivatives such as exatecan or deruxtecan), antimitotic agents (in particular auristatin derivatives such as MMAE or MMAF), cytotoxic agents (such as maytansins) and/or corticosteroids.

14. A pharmaceutical composition comprising an active agent, which is an antibody-drug conjugate according to any one of claims 1-13, and a pharmaceutically acceptable carrier and/or excipient.

15. An antibody-drug conjugate of any one of claims 1-14 or a pharmaceutical composition of claim 14 for use in medicine, particularly in human medicine.

16. An antibody-drug conjugate of any one of claims 1-13 or a pharmaceutical composition of claim 14 for use in the prevention and/or treatment of a (TCR)-CD3-complex associated disorder, particularly for the prevention and/or the treatment of inflammatory diseases, autoimmune diseases, and/or immunosuppressive induction therapy for transplant engraftment, immunosuppressive therapy in graft versus host disease and/or targeting T lymphocytes in the treatment of T-cell malignancies.
